# EUROPÄISCHE PATENTSCHRIFT

(11) **EP 3 042 605 B1**
(45) Veröffentlichungstag und Bekanntmachung des Hinweises auf die Patenterteilung: **11.11.2020**
(21) Anmeldenummer: 15201556.6
(22) Anmeldetag: 21.12.2015
(51) Int. Cl.: A61B 5/021, A61B 7/04, A61B 5/00

(54) **VERFAHREN UND VORRICHTUNG ZUR MESSUNG DER PULSWELLENGESCHWINDIGKEIT EINER MESSPERSON**
METHOD AND DEVICE FOR MEASURING PULSE WAVE VELOCITY OF A MEASURING PERSON
PROCEDE ET DISPOSITIF DESTINES A LA MESURE DE LA PRESSION SANGUINE D'UNE PERSONNE

(30) Priorität: 09.01.2015 DE 102015000328
(43) Veröffentlichungstag der Anmeldung: 13.07.2016
(73) Patentinhaber: Technische Hochschule Lübeck, 23562 Lübeck (DE)
(72) Erfinder: Ryschka, Martin, 23617 Stockelsdorf (DE); Kaufmann, Steffen, 21357 Barum (DE); Ardelt, Gunther, 22967 Tremsbüttel (DE); Malhotra, Ankit, 23562 Lübeck (DE); Kusche, Roman, 21075 Hamburg (DE)
(74) Vertreter: Wallinger, Michael

(56) Entgegenhaltungen:
- JP-A- 2004 305 268
- US-A1- 2002 107 450
- US-A1- 2006 264 755
- R. Kusche, A. Malhotra, M. Ryschka, S. Kaufmann: "A Portable In-Ear Pulse Wave Measurement System", , Oktober 2014 (2014-10), XP002757250, Gefunden im Internet: URL:https://www.researchgate.net/publicati on/281438955_A_Portable_In-Ear_Pulse_Wave_ Measurement_System [gefunden am 2016-05-02]
- STEFFEN KAUFMANN, GUNTHER ARDELT, ANKIT MALHOTRA, MARTIN RYSCHKA: "In-Ear Pulse Wave Measurements: A Pilot Study", BIOMEDICAL ENGINEERING / BIOMEDIZINISCHE TECHNIK, Nr. 58, 7. September 2013 (2013-09-07), XP002757251, ISSN: 0013-5585, DOI: 10.1515/bmt-2013-4128
- S. Kaufmann, A. Malhotra, G. Ardelt, N. Hunsche, K. Breßlein, R. Kusche, and M. Ryschka: "A System for In-Ear Pulse Wave Measurements", , Januar 2014 (2014-01), XP002757252, Gefunden im Internet: URL:http://lme.fh-luebeck.de/Joomla/images /PDF/2014%20A%20System%20for%20In-Ear%20Pu lse%20Wave%20Measurements.pdf [gefunden am 2016-05-02]
- R. KUSCHE, P. KLIMACH, A. MALHOTRA, S. KAUFMANN AND M. RYSCHKA: "An in-ear pulse wave velocity measurement system using heart sounds as time reference", CURRENT DIRECTIONS IN BIOMEDICAL ENGINEERING, Bd. 1, Nr. 1, 12. September 2015 (2015-09-12), Seiten 366-370, XP002757253, ISSN: 2364-5504, DOI: 10.1515/cdbme-2015-0090

## Beschreibung

Die Erfindung betrifft ein Verfahren zur Messung der Pulswellengeschwindigkeit einer Messperson, d. h. der Ausbreitungsgeschwindigkeit von Druckwellen im Blut des Arteriensystems der Messperson, sowie eine Messvorrichtung zur Pulswellengeschwindigkeitsmessung, insbesondere zur Durchführung dieses Verfahrens.

Ausgangspunkt für das erfindungsgemäße Verfahren und die erfindungsgemäße Messvorrichtung sind die hohe Zahl von Gefäßerkrankungen, für deren Diagnose und Prävention die Untersuchung der arteriellen Gefäßsteifigkeit mittels Pulswellenanalyse geeignet ist.

Unter anderem liefert diese Untersuchungsmethode eine prädiktive Größe für die Feststellung und Beurteilung kardiovaskulärer Ereignisse. Weiter liefert diese Untersuchungsmethode wesentliche Ergebnisse zur Bestimmung einer Therapie oder Therapieoptimierung.

Ein weiterer wesentlicher Vorteil diese Untersuchungsmethode gegenüber Methoden mit Eingriffen in den Körper ist die nicht-invasive Bestimmung des Aortendruckverlaufs und die Ableitung prädiktiver Parameter wie des Augmentationsindex und der Pulswellengeschwindigkeit (pulse wave velocity, PWV).

Von wesentlichem Interesse ist dabei die thorakale Pulswellengeschwindigkeit, welche hauptsächlich durch die Elastizität der Aorta bestimmt wird. Damit ist die Pulswellengeschwindigkeit ein deutlicher Indikator für die arterielle Gefäßsteifigkeit.

Grundsätzlich kann die Pulswellengeschwindigkeit aus der Zeitdifferenz des Auftretens der Pulswelle in einer Arterie an zwei voneinander entfernten Stellen mit einem bekannten Abstand bestimmt werden. Nicht-invasiv ist dies nur an äußerlich zugänglichen Arterien, z. B. an den Extremitäten, möglich.

Alternativ kann auch die Zeitdifferenz zwischen dem Auftreten der Pulswelle an einem geeigneten Ort, wie der Halsschlagader (Arteria carotis) oder der Speichenarterie (Arteria radialis) und natürlich am Entstehungsort im Herzen erfasst werden.

Der Anstoß der Pulswelle an die Arterienwand am Messort wird im Herzen durch die Kontraktion des Herzmuskels ausgelöst. Allgemein ist das durch das sogenannte Pulsfühlen, d. h. das Ertasten einer pulsierenden Arterie, bekannt. Geeignete Stellen für die Pulsmessung sind bekanntermaßen überall dort, wo Arterien oberflächlich verlaufen und gegen eine harte Unterlage wie Knochen oder Muskeln gedrückt werden können.

Die Zeitspanne, die die Pulswelle vom Herzen bis zu einem bestimmten Messort benötigt, wird als Pulse Arrival Time (PAT) bezeichnet.

Dabei kann der Startzeitpunkt der Pulswelle aus der sogenannten R-Zacke des EKG (Elektrokardiogramm) unter Berücksichtigung der sogenannten Pre-Ejection Period (PEP) bestimmt werden. Die PEP ist dabei die Zeitspanne von der R-Zacke des EKG (elektrische Herzaktivität) bis zum Auswurf des Blutes aus dem Herzen (mechanische Herzaktivität).

Ebenso kann die Pulswellengeschwindigkeit aus dem an einem einzigen Ort gewonnenen zeitlichen Verlauf der Pulswelle unter Annahme einer bekannten Systemfunktion des Arteriensystems berechnet werden. Diese Systemfunktion muss empirisch ermittelt werden und unterliegt großen intra- und interpersonellen Schwankungen.

Auch für eine indirekte Blutdruckmessung lässt sich die Messung der Pulswellengeschwindigkeit mit Hilfe eines EKG einsetzen, da die Pulswellenlaufzeit, also der zeitliche Abstand zwischen einer bestimmten Stelle im EKG und dem Eintreffen der Pulswelle an einer bestimmten Stelle des Körpers, wesentlich durch den Blutdruck bestimmt wird.

Bei klassischen Verfahren zur Messung der Pulswellengeschwindigkeit, bei denen als Referenz ein EKG genutzt wird, wie z. B. in der DE 24 60 839 C3 beschrieben, wird die Ankunftszeit einer Pulswelle auf optischem Wege, in der Regel durch einen Beobachter an einem Messinstrument, einem Schreiber oder einer digitalisierten Darstellung durchgeführt. Systembedingt werden dabei mindestens zwei Messorte und zwei Sensoren benötigt. Dabei erfolgt die Auswertung z. B. über die Interpretation der Morphologie der detektierten Pulswelle.

Die Dissertation von E. Stein (Medizinische Fakultät der Universität Ulm, 2008) betrifft die "Nichtinvasive Messung des intracraniellen Druckes mit Hilfe der Infraschallemission des Trommelfells". In dieser Dissertation wird der intrakranielle Druck bestimmt. Dies wird durch eine Druckmessung im abgedichteten Gehörgang mit einem herkömmlichen Drucksensor erreicht.

Die US 2014/0051940 A1 zeigt eine Messanordnung für physiologische Parameter, u. A. der Pulswellengeschwindigkeit und darüber des Blutdrucks, mit Sensoren, die in einem oder in zwei Ohrhörern integriert sind, weiteren auf der Haut aufgebrachten Elektroden sowie einem tragbaren Gerät, beispielsweise einem Mobiltelefon, zur Auswertung und Anzeige der Parameter. Als Referenz für die Erzeugung der Pulswelle dienen dabei wiederum bestimmte Spitzen im EKG. In der US 2002/107450 A1 werden Pulswellen zur Pulswellengeschwindigkeitsmessung verwendet, die durch einen Herzschlag ausgelöst werden. Die US 2006/264755 A1 beschreibt eine Messung der Pulswellengeschwindigkeit mittels Erzeugung einer Pulswelle durch eine künstliche Anregung durch Betätigung eines Aktors.

Es ist die Aufgabe der Erfindung, ein Verfahren sowie eine Messvorrichtung, insbesondere zur Durchführung dieses Verfahrens, anzugeben, durch welche eine nicht-invasive Pulswellengeschwindigkeitsmessung einfach und zuverlässig durchführbar ist.

Diese Aufgabe wird durch ein Verfahren und eine Messvorrichtung gemäß den unabhängigen Ansprüchen gelöst.

Vielen oben genannten, bekannten Methoden zur Pulswellengeschwindigkeitsmessung ist gemein, dass eine Laufzeitmessung der Pulswelle innerhalb einer bekannten Strecke mit mindestens zwei örtlich voneinander getrennten Sensoren erfolgt. Dabei erfolgt die Laufzeitmessung in der Regel vom Herzen zu den Extremitäten (z. B. zu einem Finger).

Wie oben erwähnt, wird als Startzeitpunkt der Pulswelle meist die R-Zacke des EKG genutzt. Das allgemeine Problem, das die bekannten Methoden zu bewältigen haben, ist, dass das EKG nur die elektrische Aktivität des Herzens widerspiegelt. Das tatsächliche Hinauspumpen des Blutes und somit die Ausbreitung der Pulswelle beginnt, wie oben beschrieben, aber erst nach einer variablen, unbekannten Zeitspanne PEP.

Wie die Erfinder erkannt haben, führen individuelle Schwankungen der PEP zwischen verschiedenen Messpersonen hierbei jedoch zu einer Messunsicherheit der so gewonnenen Pulswellengeschwindigkeit.

Die Erfindung basiert daher auf der Idee, durch eine Messung im Ohr oder an einer anderen geeigneten Stelle des Körpers sowohl den Startzeitpunkt der Erzeugung einer Pulswelle als auch den Ankunftszeitpunkt, an dem die Pulswelle das Ohr oder die andere geeigneten Stelle des Körpers der Messperson erreicht, durch dieselbe Druckmesseinrichtung zu bestimmen. Die Bestimmung des Startzeitpunkts erfolgt dabei über Druckänderungen im Körper der Messperson, die durch die Erzeugung der Pulswelle ausgelöst werden und sich im Vergleich zu der Pulswelle selbst sehr schnell im Körper der Messperson ausbreiten, vorzugsweise Schallwellen. Die Bestimmung des Ankunftszeitpunkts erfolgt über die im Gehörgang der Messperson oder an einer anderen geeigneten Stelle bei der Ankunft der Pulswelle auftretenden Druckänderungen.

Vorzugsweise ist bei der Messung im Ohr der Gehörgang bei der Messung abgedichtet.

Das erfindungsgemäße Verfahren zur nicht-invasiven Messung der Pulswellengeschwindigkeit einer Messperson weist die folgenden Schritte auf:
- Einführen oder Anbringen einer Druckmesseinrichtung in ein Ohr oder an einer anderen geeigneten Stelle des Körpers der Messperson,
- Messen eines zeitlichen Druckverlaufs im Gehörkanal oder an der anderen geeigneten Stelle des Körpers der Messperson durch die Druckmesseinrichtung,
- Bestimmen des Startzeitpunkts der Erzeugung einer Pulswelle an einem Pulswellenerzeugungsort im oder am Körper der Messperson, welcher vom Ort der Druckmesseinrichtung im Ohr oder der andere geeigneten Stelle des Körpers der Messperson verschieden ist und welcher einen bekannten, im arteriellen Gefäßsystem der Messperson gemessenen Abstand vom Ort oder der anderen geeigneten Stelle des Körpers der Druckmesseinrichtung hat, aus dem zeitlichen Druckverlauf,
- Bestimmen des Ankunftszeitpunkts, an dem die Pulswelle die Druckmesseinrichtung erreicht, aus dem zeitlichen Druckverlauf,
- Bestimmen der Pulswellengeschwindigkeit aus der Differenz des Ankunftszeitpunkts und des Startzeitpunkts sowie aus dem Abstand des Ortes der Druckmesseinrichtung von dem Pulswellenerzeugungsort.

Das erfindungsgemäße Verfahren ermöglicht eine einfache, zuverlässige und genaue nicht-invasive Bestimmung der Pulswellengeschwindigkeit, besonders bevorzugt durch die Anbringung eines Sensors an nur einem einzigen Messort vorzugsweise im Gehörkanal. Zusätzlich können mit der Erfindung weitere Vitalparameter bestimmt werden.

Für die Messung wird die Ankunftszeitpunkt der arteriellen Pulswelle durch die damit einhergehende Druckänderung im, vorzugsweise abgeschlossenen, Gehörkanal, ggf. zusätzlich auch durch ein im Gehörkanal gemessenes Photoplethysmogramm (PPG), durch die erfindungsgemäße Messvorrichtung bestimmt.

In einer bevorzugten Ausführung des Verfahrens ist die Pulswelle eine durch die Kontraktion des Herzens der Messperson erzeugte Pulswelle, deren Startzeitpunkt aus wenigstens einem Herzton der Messperson bestimmt wird.

Dieser Ausführung liegt die Erkenntnis zu Grunde, dass die Herztöne einen besseren Indikator für den Startzeitpunkt der Pulswelle im Herzen darstellen als beispielsweise die R-Zacke des EKG wegen der oben beschriebenen Messungenauigkeit durch die Zeitspanne PEP. Die Herztöne werden dagegen durch die mechanische Aktivität der Herzklappen erzeugt und erlauben daher eine sehr genaue Definition des Startzeitpunktes der Pulswelle. Außerdem breiten sich die Herztöne im Körper der Messperson als Körperschall mit Schallgeschwindigkeit aus. Ihre Laufzeit vom Herzen zur Druckmesseinrichtung im Ohr oder der anderen geeigneten Stelle des Körpers für die Bestimmung des Startzeitpunkts der Pulswelle kann daher vernachlässigt werden oder, vorzugsweise, aufgrund der bekannten Schallgeschwindigkeit und des bekannten Abstands im Körper der Messperson "herausgerechnet" werden.

Damit entspricht die - ggf. korrigierte - Zeitdifferenz zwischen dem über die Herztöne bestimmten Startzeitpunkt der Pulswelle und dem durch die Druckmesseinrichtung bestimmten Ankunftszeitpunkt der Pulswelle sehr genau der Pulswellenlaufzeit, woraus mit einem aus anatomischen Annahmen und/oder individuell erfassten Abstand im arteriellen Gefäßsystem vom Herzen zum Ort der Druckmesseinrichtung im Ohr oder der anderen geeigneten Stelle des Körpers die Pulswellengeschwindigkeit sehr genau bestimmt werden kann.

Der Ort der Druckmesseinrichtung im Gehörkanal ist hierfür besonders geeignet, da das Ohr dem Herzen im arteriellen Gefäßsystem relativ nahe ist. Außerdem bezieht sich die auf diese Weise gemessene Pulswellengeschwindigkeit wesentlich auf die thorakalen Arterien mit der oben erwähnten Indikatorwirkung für die Elastizität der Aorta.

Sowohl die Detektion der Pulswelle als auch die der Herztöne erfolgt dabei durch eine Messvorrichtung. Da sich die Druckmesseinrichtung vorzugsweise im Gehörkanal befindet, kann die Druckmesseinrichtung vorzugsweise als Mikrofon in einem In-Ear-Kopfhörer verwirklicht sein.

Die Druckmesseinrichtung kann aber auch in Form von anderen Sensortypen, z. B. als Photoplethysmografie-, Tonometer- oder Ultraschallsensor, welche ebenfalls als Druckmesseinrichtungen oder Drucksensoren im Sinne der vorliegenden Anmeldung verstanden werden, oder als Kombination davon für die bevorzugt simultane Erfassung der Herztöne und der Pulswelle verwirklicht sein.

In einer weiteren bevorzugten Ausgestaltung der Erfindung kann die Druckmesseinrichtung anstatt im Gehörkanal auch an einem anderen geeigneten Messort im oder am Körper der Messperson angeordnet sein, wie z. B. an der gut ertastbaren Arteria carotis oder an der Arteria temporalis.

In der bevorzugten Ausführung des erfindungsgemäßen Verfahrens wird die Pulswelle durch eine künstliche Anregung des Arteriensystems der Messperson durch die Betätigung eines Aktors erzeugt.

Hierdurch können genau definierte und reproduzierbare Pulswellen erzeugt werden.

Vorzugsweise wird durch den Aktor von außen im Arteriensystem eine künstliche Pulswelle mit geringer Amplitude erzeugt.

Der Aktor zur Pulswellenerzeugung ist vorzugsweise eine Druckmanschette, die an einer der zugänglichen Extremitäten angebracht ist. Die Druckmanschette wird vorzugsweise mit einem geeigneten konstanten Vordruck zur Entspannung der Arterienwände und einem kleinen Druckimpuls beaufschlagt.

Weiter kann ein elektromechanischer Aktor verwendet werden, der über einer Arterie auf die Haut aufgesetzt wird und einen Druckimpuls über die Haut und das Gewebe in die Arterie überträgt.

Auch ist eine künstliche Pulsanregung in einer Arterie durch eine oder mehrere, vorzugsweise geeignet gekoppelte und ausgerichtete Ultraschallwellen möglich.

In einer bevorzugten Variante dieser Ausführung des erfindungsgemäßen Verfahrens wird der gemessene zeitliche Druckverlauf in eine Mehrzahl von einzelnen Teilabschnitten, jeweils beginnend mit dem Startzeitpunkt einer Pulswelle, zerlegt, aus den einzelnen Teilabschnitte ein aggregierter Teilabschnitt, insbesondere durch Mittelwertbildung, erzeugt und der Ankunftszeitpunkt aus dem aggregierten Teilabschnitt bestimmt. Vorzugsweise erfolgt die Anregung durch den Aktor dabei periodisch.

Dadurch, dass die von dem Aktor erzeugten Pulswellen genau definiert und reproduzierbar sind, und insbesondere, wenn der Aktor und die Druckmesseinrichtung durch dieselbe Steuereinheit gesteuert werden, ist eine solche Zerlegung besonders einfach möglich. Durch diesen Verfahrensschritt können auch sehr kleine Druckamplituden in dem aggregierten Teilabschnitt aus dem Rauschen des gemessenen zeitlichen Druckverlaufs hervorgehoben werden.

In einer weiteren bevorzugten Ausführung des erfindungsgemäßen Verfahrens wird der von der Druckmesseinrichtung gemessene zeitliche Druckverlauf mit wenigstens einem Muster für einen typischen Druckverlauf verglichen.

Auf diese Weise können in einem Regelkreis die Parameter eines Signalverarbeitungs-Algorithmus laufend den aktuellen Messungen angepasst werden.

Da gerade die Herztöne mit der Entfernung zum Herzen schwächer und zunehmend von anderen Körpergeräuschen überlagert werden, werden bevorzugt auch Methoden für die Signalaufbereitung der Herztöne eingesetzt, insbesondere die Korrelation der detektierten Signale für die Herztöne und für die Pulswelle.

In einer weiteren bevorzugten Ausführung des erfindungsgemäßen Verfahrens wird die Pulswellengeschwindigkeit mehrfach hintereinander bestimmt. Auf diese Weise wird ein, vorzugsweise quasi-kontinuierlicher, zeitlicher Verlauf der Pulswellengeschwindigkeit bestimmt.

Dadurch wird eine hohe, ungestörte Messgenauigkeit erreicht. Außerdem sind Langzeitmessungen oder Fitnesskontrollen einfach und ohne Beeinträchtigung der Bewegungsfreiheit und damit der Lebensqualität der Messperson, insbesondere eines Sportlers, möglich.

Weiterhin wird dadurch die Detektion von weiteren Körperfunktionen ermöglicht, für welche ein bestimmter zeitlicher Verlauf der Pulswellengeschwindigkeit typisch ist.

In einer bevorzugten Variante dieser Ausführung des erfindungsgemäßen Verfahrens wird aus dem so bestimmten zeitlichen Verlauf der Pulswellengeschwindigkeit wenigstens ein Schluckvorgang und/oder wenigstens ein Atemvorgang der Messperson detektiert.

In einer weiteren bevorzugten Variante dieser Ausführung des erfindungsgemäßen Verfahrens wird aus dem so bestimmten Verlauf der Pulswellengeschwindigkeit der momentane Blutdruck und/oder der zeitliche Verlauf des Blutdrucks der Messperson detektiert.

Die Blutdruckmessung kann dabei ohne Manschette erfolgen, sondern allein durch die Bestimmung des Blutdrucks aus dessen Abhängigkeit von der Pulswellengeschwindigkeit.

Eine erfindungsgemäße Messvorrichtung zur Pulswellengeschwindigkeitsmessung, insbesondere zur Durchführung des erfindungsgemäßen Verfahrens, weist die folgenden Komponenten auf:
- eine Druckmesseinrichtung, welche in ein Ohr der Messperson oder an einer anderen geeigneten Stelle des Körpers einführbar oder anbringbar ist, zur Messung des zeitlichen Druckverlaufs im Gehörkanal oder an dieser anderen geeigneten Stelle des Körpers der Messperson,
- eine erste Bestimmungseinrichtung zur Bestimmung des Startzeitpunkts der Erzeugung einer Pulswelle an einem Pulswellenerzeugungsort im oder am Körper der Messperson, welcher vom Ort der Druckmesseinrichtung im Ohr oder der anderen geeigneten Stelle des Körpers der Messperson verschieden ist, aus dem zeitlichen Druckverlauf
- eine zweite Bestimmungseinrichtung zur Bestimmung des Ankunftszeitpunkts, an dem diese Pulswelle die Druckmesseinrichtung erreicht, aus dem zeitlichen Druckverlauf,
- eine dritte Bestimmungseinrichtung zur Bestimmung der Pulswellenlaufzeit aus den beiden bestimmten Zeitpunkten,
- eine vierte Bestimmungseinrichtung zur Bestimmung der Pulswellengeschwindigkeit aus der Pulswellenlaufzeit und dem Abstand des Ortes der Druckmesseinrichtung von dem Pulswellenerzeugungsort.

Selbstverständlich können auch mehrere der ersten bis vierten Bestimmungseinrichtung auch in einzelnen Hardware- und/oder Softwaremodulen gemeinsam verwirklicht sein, oder es kann sich umgekehrt die Funktionalität einer Bestimmungseinrichtung auf mehrere solche Module verteilen.

Die Anbringung und Anwendung der erfindungsgemäßen Messvorrichtung ist ohne spezielle Schulung auch durch die Messperson selbst möglich. Damit wird eine ambulante Pulswellengeschwindigkeitsmessung stark vereinfacht.

Beim Einsatz der Messvorrichtung während einer Operation wird zudem die Bewegungsfreiheit des Arztes kaum eingeschränkt.

In einer bevorzugten Ausführung der erfindungsgemäßen Messvorrichtung weist die Druckmesseinrichtung wenigstens einen Drucksensor, vorzugsweise genau einen Drucksensor, auf.

Wenn die Druckmesseinrichtung genau einen Drucksensor aufweist, können sowohl der Startzeitpunkt als auch der Ankunftszeitpunkt der Pulswelle aus dem von diesem einen Sensor gemessenen zeitlichen Druckverlauf bestimmt werden, wodurch die Druckmesseinrichtung besonders klein gestaltet und besonders kostengünstig hergestellt werden kann.

In einer bevorzugten Variante dieser Ausführung der erfindungsgemäßen Messvorrichtung ist die Druckmesseinrichtung derart ausgelegt, dass aus dem von ihr gemessenen zeitlichen Druckverlauf sowohl niederfrequente Druckänderungen, vorzugsweise im Bereich der menschlichen Herzfrequenz, insbesondere im Bereich von 20 bis 220 min⁻¹, als auch hörbarer Schall, vorzugsweise im Bereich der Schallfrequenz der menschlichen Herztöne, insbesondere im Bereich von 15 bis 400 Hz, detektiert werden kann.

Auf diese Weise können sowohl die niederfrequenten Druckänderungen der Pulswelle als auch der hörbare Schall der Herztöne von demselben Sensor detektiert werden.

Bevorzugt weist die Messeinrichtung einen Photoplethysmografie-(PPG)-Sensor auf, welcher ebenfalls in ein Ohr der Messperson einführbar ist und welcher die Ankunft einer Pulswelle im Ohr optisch detektieren und/oder eine SpO₂-Messung vornehmen kann.

In einer weiteren bevorzugten Ausführung weist die erfindungsgemäße Messvorrichtung weiterhin einen Beschleunigungssensor, vorzugsweise zur Bestimmung einer relativen, individuellen Position (beispielsweise Stehen, Sitzen, Liegen) und/oder einer Bewegung der Messperson und/oder zur Realisierung eines Schrittzählers für Fitnessanwendungen, und/oder einen Temperatursensor zur Messung der Körpertemperatur der Messperson auf.

Auf diese Weise können auch Vitalparameter, die nicht oder nicht allein aus der Pulswellengeschwindigkeit ableitbar sind, in die Auswertung der Messergebnisse einbezogen werden, wodurch sich die Anzahl der durch die Messvorrichtung detektierbaren Körperfunktionen noch stark erhöht.

Bevorzugt wird die im Ohr positionierte Sensorik gleichzeitig als Elektrode genutzt, insbesondere für Bioimpedanz-, EKG- oder EEG-Messungen.

In einer weiteren bevorzugten Ausführung weist die erfindungsgemäße Messvorrichtung weiterhin einen Lautsprecher auf, welcher gemeinsam mit der Druckmesseinrichtung in ein Ohr der Messperson einführbar ist. Über den Lautsprecher können insbesondere Umgebungsschall, Musik, Sprache und/oder über eine Schnittstelle von einer anderen Kommunikationseinrichtung empfangene Audiosignale wiedergegeben werden.

Die Kommunikation und der Datenaustausch mit der anderen Kommunikationseinrichtung erfolgt dabei bevorzugt über eine drahtlose Schnittstelle, wie Bluetooth oder WLAN.

Auf diese Weise kann die Messperson beispielsweise trotz eines durch die Messvorrichtung abgedichteten Gehörgangs die Umgebungsgeräusche hören, oder die Messperson kann während der Messung unterhalten oder abgelenkt werden oder, insbesondere bei einem mobilen Einsatz der Messvorrichtung, mit anderen Personen drahtlos telefonieren.

Weiterhin kann die Messvorrichtung den störenden Umgebungsschall durch eine geeignete Signalverarbeitung, insbesondere durch eine Überlagerung mit entsprechendem Gegenschall, weitgehend oder vollständig eliminieren.

In der bevorzugten Ausführung weist die erfindungsgemäße Messvorrichtung weiterhin einen Aktor zur Erzeugung einer Pulswelle durch eine künstliche Anregung des Arteriensystems der Messperson auf, insbesondere eine Druckmanschette, insbesondere zur Anbringung an einer Extremität der Messperson, einen elektromechanischen Aktor oder einen Ultraschallgeber zur Einleitung eines Druckimpulses in eine Arterie der Messperson.

Durch derartige Aktoren können die oben genannten genau definierten und reproduzierbaren Pulswellen erzeugt werden.

Weitere Vorteile, Merkmale und Anwendungsmöglichkeiten der vorliegenden Erfindung ergeben sich aus der nachfolgenden Beschreibung in Zusammenhang mit den Figuren. Es zeigen:
- Fig. 1: eine Darstellung von Messergebnissen einer erfindungsgemäßen Messvorrichtung für die Pulswelle und für die Herztöne sowie von EKG-Messwerten zum Vergleich.
- Fig. 2: eine Schnittansicht einer erfindungsgemäßen Messvorrichtung in einer Variante mit Kabelverbindung.
- Fig. 3: ein Blockschaubild für eine Ausführung einer Signalverarbeitung in dem erfindungsgemäßen Verfahren.

Wie oben beschrieben, basiert die Erfindung auf dem Ansatz, dass die Laufzeit der Pulswelle vom Herzen zum Ohr oder einer anderen geeigneten Stelle des Körpers gemessen wird. Nachfolgend wird die Erfindung am Beispiel der In-Ohr-Messung beschrieben. Diese Beschreibung ist aber nicht als einschränkend für die Anwendung der Erfindung zu verstehen.

Bei der In-Ohr-Messung wird die Ankunft der Pulswelle durch die auftretenden Druckänderungen in einem, vorzugsweise abgedichteten, Gehörgang detektiert.

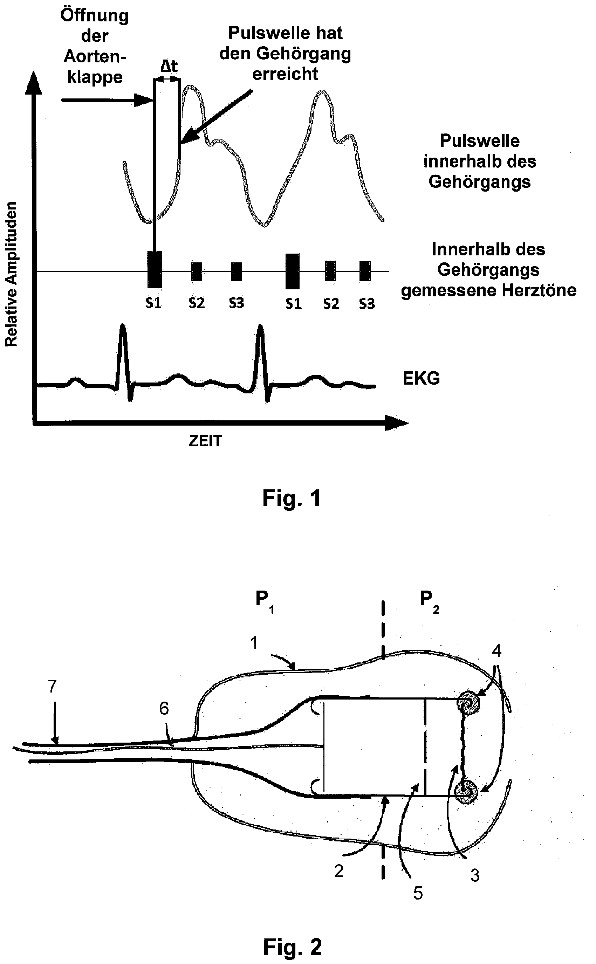

Fig. 1 zeigt in der oberen Messkurve den zeitlichen Verlauf einer Druckwelle im Gehörkanal. Die mittlere Darstellung zeigt den im Gehörkanal gemessenen zeitlichen Verlauf der Herztöne. Die Herztöne sind dabei, entsprechend der allgemein üblichen Bezeichnung, mit S1, S2 und S3 kenntlich gemacht. Da die Herztöne durch die mechanische Aktivität der Herzklappen erzeugt werden, erlauben sie eine sehr genaue Definition des Startzeitpunktes der Pulswelle. Die untere Messkurve zeigt einen Referenz-EKG-Signalverlauf.

Das mit Pfeilen gekennzeichnete Intervall Δt steht für die gemessene Pulswellenlaufzeit. Die Amplituden der gezeigten Kurven bzw. der detektierten Ereignisse sind zueinander lediglich relativ dargestellt.

In dem in Fig. 2 dargestellten Ausführungsbeispiel einer erfindungsgemäßen Messvorrichtung ist diese funktionell als ein einziger Sensor ausgebildet, durch den sowohl der Startzeitpunkt der Pulswelle als auch der Ankunftszeitpunkt der Pulswelle im Ohr detektierbar ist. Dabei besteht die Sensorik aus einem Mikrofon, durch das auch eine niederfrequente Druckänderung detektierbar ist.

Fig. 2 zeigt den Aufbau einer Variante der erfindungsgemäßen Messvorrichtung im Schnittmodell. Die Messvorrichtung zur In-Ohr-Messung weist dabei eine Ohrolive 1, z. B. eine Stethoskop-Ohrolive, auf, die sich an die Form des Gehörgangs anpassen lässt, diesen druckdicht abschließt und die Messvorrichtung im Ohr positioniert. Die Ohrolive 1 ist in Richtung Gehörgang (auf der rechten Seite) offen, um einen direkten Zugang zu einem Sensor zu ermöglichen. In die Ohrolive 1 ist als Sensor eine Mikrofonkapsel 2 eingebracht, die in Richtung des Gehörgangs eine Schallmembran 3 aufweist. Die Schallmembran 3 ist in der Mikrofonkapsel 2 aufgehängt und durch Abdichtungen 4 mit dieser verbunden. Weiter weist die Mikrofonkapsel 2 eine Gegenelektrode 5 auf. In der gezeigten Variante besteht die Schnittstelle zu einer Signalverarbeitungseinheit (nicht dargestellt) in einer Kabelverbindung, wobei ein Kabel 6, welches die Messsignale überträgt, in einem Schrumpfschlauch 7 verläuft, der die Mikrofonkapsel 2 fest umschließt.

Durch den druckdichten Abschluss des Gehörgangs durch die Ohrolive 1 werden zumindest zwei Zonen mit zueinander unterschiedlichen Druckverläufen gebildet. In der ersten Zone herrscht der Umgebungsdruck P₁, der außerhalb der Ohrolive 1 und innerhalb der Mikrofonkapsel 2 an der Schallmembran 3 anliegt. In der zweiten Zone herrscht der Druck P₂ im Gehörgang und in der Ohrolive 1, der außen an der Schallmembran 3 anliegt.

Eine erfindungsgemäße Messvorrichtung beschränkt sich nicht auf den in Fig. 1 gezeigten Aufbau. Im Bedarfsfall sind neben der gezeigten Sensorik weitere Sensoren und/oder Aktoren in die Ohrolive 1 integrierbar. Die Schnittstelle zur Signal- und/oder Datenübertragung kann durch eine Kabelverbindung und/oder kabellos, digital oder auch analog, erfolgen. Damit ist es auch möglich, die Signalverarbeitung mit der gesamten erforderlichen Hard- und Software in die Ohrolive 1 bzw. in die Sensorik zu integrieren.

Die Energieversorgung der Messvorrichtung kann auch auf dem Wege des "human-powered energy harvesting" durch den menschlichen Körper direkt erfolgen.

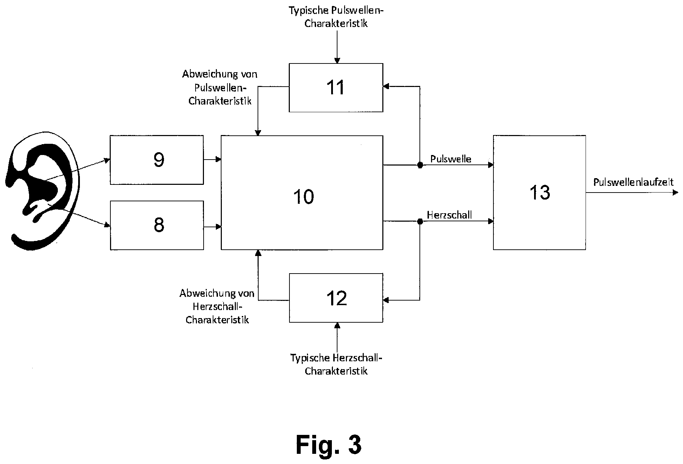

Fig. 3 zeigt beispielhaft ein Blockdiagramm für die Signalverarbeitung in dem erfindungsgemäßen Verfahren zur In-Ohr-Messung mit internen Regelkreisen, in denen die Parameter für die Signalverarbeitung stets den aktuellen Messungen angepasst werden. Das Ausgangssignal ist dabei die Pulswellenlaufzeit als Messwert, der zur Darstellung, Speicherung, Verarbeitung oder dergleichen ausgegeben wird.

Das Blockdiagramm zeigt die Übergabe von Druckverlaufs- und anderen Messwerten, die durch die Druckmesseinrichtung 8 sowie ggf. durch weitere Sensoren 9, beispielsweise durch einen PPG-Sensor, erfasst wurden, an ein Modul Signalverarbeitung 10. Das Modul Signalverarbeitung 10 prozessiert die quasikontinuierlichen Messwerte der Sensorik, um die zeitabhängigen Sensorsignale in zwei Signale für den Herzschall bzw. für die Pulswelle zu trennen und einer Differenzzeit-Berechnung 13 zuzuführen, welche die Pulswellenlaufzeit als Ausgangsgröße berechnet.

Weiterhin weist die Signalverarbeitung zwei Regelkreise auf mit einem Vergleicher 12, welcher eine typische Herzschall-Charakteristik mit der gemessenen Herzschall-Charakteristik vergleicht, sowie einen Regelkreis mit einem Vergleicher 11, welcher eine typische Pulswellen-Charakteristik mit der gemessenen Pulswellen-Charakteristik vergleicht, und jeweils die Abweichungen an das Modul Signalverarbeitung 10 zurückgibt, damit diese ihre interne Parameter anpassen kann, um die Trennung sowie die Qualität der beiden Signale zu verbessern.

Die sich aus der Differenzzeit-Berechnung 13 ergebende Pulswellenlaufzeit wird als Wert zur Darstellung und zur weiteren Verarbeitung ausgegeben.

### Bezugszeichenliste

- 1: Ohrolive
- 2: Mikrofonkapsel
- 3: Schallmembran
- 4: Abdichtung
- 5: Gegenelektrode
- 6: Kabel
- 7: Schrumpfschlauch

- P₁: Umgebungsdruck
- P₂: Druck im Gehörgang

- 8: Druckmesseinrichtung
- 9: Weitere Sensoren
- 10: Modul Signalverarbeitung
- 11: Vergleicher für Pulswellen-Charakteristik
- 12: Vergleicher für Herzschall-Charakteristik
- 13: Differenzzeit-Berechnung

## Patentansprüche

1. Verfahren zur nicht-invasiven Messung der Pulswellengeschwindigkeit einer Messperson, d. h. der Ausbreitungsgeschwindigkeit von Druckwellen im Blut des Arteriensystems der Messperson, mit den Schritten:
- Einführen und/oder Anbringung einer Druckmesseinrichtung (8) in einem Ohr oder in bzw. an einer anderen geeigneten Stelle des Körpers der Messperson,
- Erzeugen einer Pulswelle durch eine künstliche Anregung des Arteriensystems der Messperson durch die Betätigung eines Aktors,
- Messen eines zeitlichen Druckverlaufs im Gehörkanal oder an der anderen geeigneten Stelle des Körpers der Messperson durch die Druckmesseinrichtung (8),
- Bestimmen, mittels einer Bestimmungseinrichtung, des Startzeitpunkts der Erzeugung der Pulswelle an einem Pulswellenerzeugungsort im oder am Körper der Messperson, welcher vom Ort der Druckmesseinrichtung (8) der Messperson verschieden ist und welcher einen bekannten, im arteriellen Gefäßsystem der Messperson gemessenen Abstand vom Ort der Druckmesseinrichtung (8) hat, aus dem zeitlichen Druckverlauf,
- Bestimmen, mittels einer weiteren Bestimmungseinrichtung, des Ankunftszeitpunkts, an dem die Pulswelle die Druckmesseinrichtung (8) erreicht, aus dem zeitlichen Druckverlauf,
- Bestimmen, mittels einer weiteren Bestimmungseinrichtung, der Pulswellengeschwindigkeit insbesondere aus der Differenz des Ankunftszeitpunkts und des Startzeitpunkts sowie aus dem Abstand des Ortes der Druckmesseinrichtung (8) von dem Pulswellenerzeugungsort.

2. Verfahren nach Anspruch 1, **dadurch gekennzeichnet, dass** der gemessene zeitliche Druckverlauf in eine Mehrzahl von einzelnen Teilabschnitten, jeweils beginnend mit dem Startzeitpunkt einer Pulswelle, zerlegt, aus den einzelnen Teilabschnitten ein aggregierter Teilabschnitt, insbesondere durch Mittelwertbildung, erzeugt und der Ankunftszeitpunkt aus dem aggregierten Teilabschnitt bestimmt wird.

3. Verfahren nach einem der vorhergehenden Ansprüche, **dadurch gekennzeichnet, dass** der von der Druckmesseinrichtung (8) gemessene zeitliche Druckverlauf mit wenigstens einem Muster für einen typischen Druckverlauf verglichen wird.

4. Verfahren nach einem der vorhergehenden Ansprüche, **dadurch gekennzeichnet, dass** die Pulswellengeschwindigkeit mehrfach hintereinander bestimmt wird, und dass auf diese Weise ein, vorzugsweise quasi-kontinuierlicher, zeitlicher Verlauf der Pulswellengeschwindigkeit bestimmt wird.

5. Verfahren nach Anspruch 4, **dadurch gekennzeichnet, dass** aus dem bestimmten zeitlichen Verlauf der Pulswellengeschwindigkeit wenigstens ein Schluckvorgang und/oder wenigstens ein Atemvorgang der Messperson detektiert wird.

6. Verfahren nach Anspruch 4 oder 5, **dadurch gekennzeichnet, dass** aus dem bestimmten Verlauf der Pulswellengeschwindigkeit der momentane Blutdruck und/oder der zeitliche Verlauf des Blutdrucks der Messperson detektiert wird.

7. Messvorrichtung zur Pulswellengeschwindigkeitsmessung, insbesondere zur Durchführung des Verfahrens nach einem der vorhergehenden Ansprüche, mit:
- einer Druckmesseinrichtung (8), welche in ein Ohr der Messperson einführbar ist oder an einer anderen geeigneten Stelle des Körpers einführbar und/oder anbringbar ist, zur Messung des zeitlichen Druckverlaufs im Gehörkanal oder an der anderen geeigneten Stelle des Körpers der Messperson,
- einem Aktor zur Erzeugung einer Pulswelle durch eine künstliche Anregung des Arteriensystems der Messperson,
- einer ersten Bestimmungseinrichtung (10, 12) zur Bestimmung des Startzeitpunkts der Erzeugung der Pulswelle an einem Pulswellenerzeugungsort im oder am Körper der Messperson, welcher vom Ort der Druckmesseinrichtung (8) im Ohr der Messperson oder an der anderen geeigneten Stelle des Körpers verschieden ist, aus dem zeitlichen Druckverlauf,
- einer zweiten Bestimmungseinrichtung (10, 11) zur Bestimmung des Ankunftszeitpunkts, an dem diese Pulswelle die Druckmesseinrichtung (8) erreicht, aus dem zeitlichen Druckverlauf,
- einer dritten Bestimmungseinrichtung (13) zur Bestimmung der Pulswellenlaufzeit aus den beiden bestimmten Zeitpunkten,
- einer vierten Bestimmungseinrichtung zur Bestimmung der Pulswellengeschwindigkeit aus der Pulswellenlaufzeit und dem Abstand des Ortes der Druckmesseinrichtung (8) von dem Pulswellenerzeugungsort.

8. Messvorrichtung nach Anspruch 7, **dadurch gekennzeichnet, dass** die Druckmesseinrichtung (8) wenigstens einen Drucksensor (2), vorzugsweise genau einen Drucksensor (2), aufweist.

9. Messvorrichtung nach Anspruch 7 oder 8, **dadurch gekennzeichnet, dass** die Druckmesseinrichtung (8) derart ausgelegt ist, dass aus dem von ihr gemessenen zeitlichen Druckverlauf sowohl niederfrequente Druckänderungen als auch hörbarer Schall detektiert werden kann.

10. Messvorrichtung nach einem der Ansprüche 7 bis 9, **dadurch gekennzeichnet, dass** sie weiterhin einen Beschleunigungssensor (9) und/oder einen Temperatursensor (9) aufweist.

11. Messvorrichtung nach einem der Ansprüche 7 bis 10, **dadurch gekennzeichnet, dass** die Druckmesseinrichtung (8) in ein Ohr der Messperson einführbar ist und dass die Messvorrichtung weiterhin einen Lautsprecher aufweist, welcher gemeinsam mit der Druckmesseinrichtung (8) in das Ohr der Messperson einführbar ist.

## Claims

1. Method for non-invasively measuring the pulse wave velocity of a measurement person, i.e. the speed of propagation of pressure waves in the blood of the arterial system of the measurement person, with the steps:
- Inserting and/or attaching a pressure measuring device (8) in an ear or in or at another suitable location of the body of the measurement person,
- generating a pulse wave by artificially stimulating the arterial system of the measurement person by actuating an actuator,
- measuring a temporal pressure curve in the auditory canal or at the other suitable location of the body of the measuring person by the pressure measuring device (8),
- determining, by means of a determining device, the start time of the generation of the pulse wave at a pulse wave generation location in or on the body of the measurement person, which is different from the location of the pressure measuring device (8) of the measurement person and which is at a known distance, measured in the arterial vascular system of the measurement person, from the location of the pressure measuring device (8), from the temporal pressure curve,
- determining, by means of a further determining device, the time of arrival at which the pulse wave reaches the pressure measuring device (8), from the temporal pressure curve,
- determining, by means of a further determining device, the pulse wave speed, in particular from the difference between the time of arrival and the start time as well as from the distance between the location of the pressure measuring device (8) and the pulse wave generation location.

2. Method according to claim 1, **characterized in that** the measured temporal pressure curve is broken down into a plurality of individual sections, each beginning with the start time of a pulse wave, an aggregated section is generated from the individual sections, in particular by averaging, and the time of arrival is determined from the aggregated section.

3. Method according to one of the preceding claims, **characterized in that** the temporal pressure curve measured by the pressure measuring device (8) is compared with at least one pattern for a typical pressure curve.

4. Method according to one of the preceding claims, **characterized in that** the pulse wave velocity is determined several times in succession, and that in this way a, preferably quasi-continuous, temporal course of the pulse wave velocity is determined.

5. Method according to claim 4, **characterized in that** at least one swallowing process and/or at least one breathing process of the measurement person is detected from the determined temporal course of the pulse wave velocity.

6. Method according to Claim 4 or 5, **characterized in that** the current blood pressure and/or the temporal course of the blood pressure of the measurement person is detected from the determined course of the pulse wave velocity.

7. Measuring device for pulse wave velocity measurement, in particular for carrying out the method according to one of the preceding claims, with:
- a pressure measuring device (8), which can be inserted into an ear of the measurement person or can be inserted and/or attached at another suitable location of the body, for measuring the temporal pressure curve in the auditory canal or at the other suitable location of the body of the measurementperson,
- an actuator for generating a pulse wave by artificially stimulating the arterial system of the measurement person,
- a first determinining device (10, 12) for determining the start time of the generation of the pulse wave at a pulse wave generation location in or on the body of the measurement person, which is different from the location of the pressure measuring device (8) in the ear of the measurement person or at the other suitable location of the body, from the temporal pressure curve,
- a second determining device (10, 11) for determining the time of arrival at which this pulse wave reaches the pressure measuring device (8), from the temporal pressure curve,
- a third determining device (13) for determining the pulse wave transit time from the two determined points of time,
- a fourth determining device for determining the pulse wave velocity from the pulse wave transit time and the distance of the location of the pressure measuring device (8) from the pulse wave generation location.

8. Measuring device according to claim 7, **characterized in that** the pressure measuring device (8) has at least one pressure sensor (2), preferably exactly one pressure sensor (2).

9. Measuring device according to claim 7 or 8, **characterized in that** the pressure measuring device (8) is designed such that both low-frequency pressure changes and audible sound can be detected from the temporal pressure curve measured by it.

10. Measuring device according to one of claims 7 to 9, **characterized in that** it further comprises an acceleration sensor (9) and/or a temperature sensor (9).

11. Measuring device according to one of claims 7 to 10, **characterized in that** the pressure measuring device (8) can be inserted into an ear of the measurement person and that the measuring device further comprises a loudspeaker which can be inserted into the ear of the measurement person together with the pressure measuring device (8).

## Revendications

1. Procédé de mesure non invasive de la vitesse d'onde puisée d'un sujet de mesure, c'est-à-dire de la vitesse de propagation d'ondes de pression dans le sang du système artériel du sujet de mesure, avec les étapes :
- d'introduction et/ou d'installation d'un système de mesure de pression (8) dans une oreille ou dans ou au niveau d'un autre emplacement adapté du corps du sujet de mesure,
- de génération d'une onde puisée par une excitation artificielle du système artériel du sujet de mesure par l'actionnement d'un actionneur,
- de mesure d'une évolution de pression dans le temps dans le conduit auditif ou au niveau de l'autre emplacement adapté du corps su sujet de mesure par le système de mesure de pression (8),
- de définition, au moyen d'un système de définition, du moment de lancement de la génération de l'onde pulsée au niveau d'un emplacement de génération d'onde pulsée dans ou au niveau du corps du sujet de mesure, lequel est différent de l'emplacement du système de mesure de pression (8) du sujet de mesure et lequel a une distance connue, mesurée dans le système vasculaire artériel du sujet de mesure, par rapport à l'emplacement du système de mesure de pression (8), à partir de l'évolution de pression dans le temps,
- de définition, au moyen d'un autre système de définition, du moment d'arrivée, auquel l'onde pulsée atteint le système de mesure de pression (8), à partir de l'évolution de pression dans le temps,
- de définition, au moyen d'un autre système de définition, de la vitesse d'onde pulsée en particulier à partir de la différence entre le moment d'arrivée et le moment de lancement ainsi qu'à partir de la distance de l'emplacement du système de mesure de pression (8) par rapport à l'emplacement de génération d'onde pulsée.

2. Procédé selon la revendication 1, **caractérisé en ce que** l'évolution de pression dans le temps mesurée est décomposée en une multitude de diverses portions partielles, respectivement commençant avec le moment de lancement d'une onde pulsée, une portion partielle agrégée est générée à partir des diverses portions partielles, en particulier par obtention d'une valeur moyenne et le moment d'arrivée est défini à partir de la portion partielle agrégée.

3. Procédé selon l'une quelconque des revendications précédentes, **caractérisé en ce que** l'évolution de pression dans le temps mesurée par le système de mesure de pression (8) est comparée à au moins un schéma pour une évolution de pression typique.

4. Procédé selon l'une quelconque des revendications précédentes, caractérisé en ce la vitesse d'onde pulsée est définie à maintes reprises l'une après l'autre, et qu'ainsi une évolution dans le temps, de préférence quasi continue, de la vitesse d'onde pulsée est définie.

5. Procédé selon la revendication 4, **caractérisé en ce qu'**au moins un processus de déglutition et/ou au moins un processus de respiration du sujet de mesure sont détectés à partir de l'évolution dans le temps définie de la vitesse d'onde pulsée.

6. Procédé selon la revendication 4 ou 5, **caractérisé en ce que** la pression artérielle instantanée et/ou l'évolution dans le temps de la pression artérielle du sujet de mesure sont détectées à partir de l'évolution définie de la vitesse d'onde pulsée.

7. Dispositif de mesure servant à la mesure de vitesse d'onde pulsée, en particulier servant à la mise en œuvre du procédé selon l'une quelconque des revendications précédentes, avec :
- un système de mesure de pression (8), lequel peut être introduit dans une oreille du sujet de mesure ou peut être introduit et/ou installé au niveau d'un autre emplacement adapté du corps, servant à la mesure de l'évolution de pression dans le temps dans le conduit auditif ou au niveau de l'autre emplacement adapté du corps du sujet de mesure,
- un actionneur servant à la génération d'une onde pulsée par une excitation artificielle du système artériel du sujet de mesure,
- un premier système de définition (10, 12) servant à la définition du moment de lancement de la génération de l'onde pulsée au niveau d'un emplacement de génération d'onde pulsée dans ou au niveau du corps du sujet de mesure, lequel est différent de l'emplacement du système de mesure de pression (8) dans l'oreille du sujet de mesure ou au niveau de l'autre emplacement adapté du corps, à partir de l'évolution de pression dans le temps,
- un deuxième système de définition (10, 11) servant à la définition du moment d'arrivée, auquel ladite onde pulsée atteint le système de mesure de pression (8), à partir de l'évolution de pression dans le temps,
- un troisième système de définition (13) servant à la définition du temps de propagation d'onde pulsée à partir des deux moments définis,
- un quatrième système de définition servant à la définition de la vitesse d'onde pulsée à partir du temps de propagation d'onde pulsée et de la distance de l'emplacement du système de mesure de pression (8) par rapport à l'emplacement de génération d'onde pulsée.

8. Dispositif de mesure selon la revendication 7, **caractérisé en ce que** le système de mesure de pression (8) présente au moins un capteur de pression (2), de préférence précisément un capteur de pression (2).

9. Dispositif de mesure selon la revendication 7 ou 8, **caractérisé en ce que** le système de mesure de pression (8) est configuré de telle manière qu'à la fois des variations de pression à basse fréquence et des sons audibles peuvent être détectés à partir de l'évolution de pression dans le temps mesurée par celui-ci.

10. Dispositif de mesure selon l'une quelconque des revendications 7 à 9, **caractérisé en ce qu'**il présente par railleurs un capteur d'accélération (9) et/ou un capteur de température (9).

11. Dispositif de mesure selon l'une quelconque des revendications 7 à 10, **caractérisé en ce que** le système de mesure de pression (8) peut être introduit dans une oreille du sujet de mesure et que le dispositif de mesure présente par ailleurs un haut-parleur, lequel peut être introduit conjointement avec le système de mesure de pression (8) dans l'oreille du sujet de mesure.
